# EUROPÄISCHE PATENTSCHRIFT

(11) **EP 2 640 410 B1**
(45) Veröffentlichungstag und Bekanntmachung des Hinweises auf die Patenterteilung: **29.10.2014**
(21) Anmeldenummer: 11785277.2
(22) Anmeldetag: 15.11.2011
(51) Int. Cl.: A61K 38/19, A61K 45/06, A61P 31/16

(54) **ZUSAMMENSETZUNG UMFASSEND EIN PEPTID UND EIN HEMMSTOFF DER VIRALEN NEURAMINIDASE**
COMPOSITION COMPRISING A PEPTIDE AND AN INHIBITOR OF VIRAL NEURAMINIDASE
COMPOSITION COMPRENANT UN PEPTIDE ET UN INHIBITEUR DE LA NEURAMINIDASE VIRALE

(30) Priorität: 18.11.2010 AT 19082010
(43) Veröffentlichungstag der Anmeldung: 25.09.2013
(73) Patentinhaber: Apeptico Forschung und Entwicklung GmbH, 1150 Wien (AT)
(72) Erfinder: FISCHER, Bernhard, A-1160 Wien (AT); LUCAS, Rudolf, B-2630 Aartselaar (BE); FISCHER, Hendrik, A-1160 Wien (AT)
(74) Vertreter: Sonn & Partner Patentanwälte
(86) Internationale Anmeldenummer: PCT/AT2011/000462
(87) Internationale Veröffentlichungsnummer: WO 2012/065201

(56) Entgegenhaltungen:
- EP-B1- 1 264 599
- WO-A1-2010/099556
- WO-A2-03/026567
- AT-A1- 506 150
- US-A1- 2008 063 722

## Beschreibung

Die vorliegende Erfindung betrifft pharmazeutische Zusammensetzungen zur Behandlung der Influenza.

Beim Menschen ist die Influenza eine durch Influenzaviren verursachte schwere Erkrankung der Atemwege und des gesamten Organismus. Die Influenzaviren gehören zur Familie der Orthomyxoviren, die durch ein segmentiertes RNA-Genom in Negativstrangorientierung gekennzeichnet sind. Die human relevanten Vertreter sind die Influenza-A und -B Viren, wobei insbesondere der Subtyp A als Erreger von hochfieberhaften Erkrankungen der Atemwege bekannt ist. Neben der tiermedizinischen Bedeutung besitzen alle Influenzaviren ein zoonitisches Potential, d.h. eine Übertragung vom Huhn oder Schwein auf den Menschen ist möglich.

Die Influenza tritt periodisch als Pandemie auf, die meist in Südostasien und China ihren Ursprung nimmt und sich von dort weltweit ausbreitet. Influenzavirenpandemien sind mit einer hohen Zahl von Todesfällen nicht nur bei älteren Personen, sondern auch bei Jugendlichen verbunden. Laut WHO resultieren aus der jährlichen saisonalen Influenza weltweit etwa 3 bis 5 Millionen Fälle von schwerer Erkrankung, mit einer Todesrate von 250000 bis 500000. Häufigste Todesursache ist die Influenzapneumonie mit resultierendem Lungenversagen, aber auch Herz-Kreislauf-Schädigungen wie Myokarditis (Herzmuskelentzündung) oder Perikarditis (Herzbeutelentzündung) kommen vor. Auch eine Entzündung des Gehirns bzw. der Gehirnhäute (Meningoencephalitis) oder die Schädigung anderer Organsysteme (insbesondere Niere) kommen als häufigere Todesursachen in Frage.

Die Inkubationszeit beträgt normalerweise 4-5 Tage, aber auch kürzer. Die Erkrankung beginnt mit dem plötzlichen Einsetzen von Kopfschmerzen, Kältegefühl, Schüttelfrost und Husten. Hohes Fieber bis 41°C, Muskelschmerzen, Appetitlosigkeit und allgemeines Schwächegefühl folgen. Diese Phase dauert etwa 3 Tage, danach geht das Fieber zurück und ist ab dem sechsten Tag meist auf normale Werte abgesunken, das Virus ist aus dem Körper eliminiert. Der Husten kann mehrere Wochen lang anhalten.

Eine schwere, lebensbedrohende Influenza tritt dann auf, wenn sich im Anschluss an die beschriebenen Symptome eine primäre virale interstitielle (oft hämorrhagische) Lungenentzündung entwickelt. Sie tritt neben geschwächten Personen auch bei etwa 25% der gesunden oder nicht vorgeschädigten Personen auf, und kann bis zu 2 Wochen dauern. Eine solche Lungenentzündung kann durch die Zunahme des Lungengewichtes nachgewiesen werden.

Lungenentzündungen können auch sekundär durch bakterielle Überinfektion (u.a. durch Streptococcus pneumoniae, Staphylococcus aureus und Haemophilus influenzae) entstehen. Begünstigende Faktoren für diese Komplikationen sind u.a. andere Lungenerkrankungen (z.B. Asthma), Immunschwäche, Alter (Kleinkinder und ältere Personen), Diabetes, Lungenverletzungen, Rauchen. Personen mit diesen Komplikationen sind daher die vorrangige Zielgruppe für die Vakzinierung.

Influenzaviren gelangen durch Tröpfcheninfektion in den Organismus und infizieren durch Bindung des HA-Proteins an endständige Neuraminsäurereste auf den Epithelzellen der Mund-, Nasen und Rachenschleimhaut. Von hier aus breiten sie sich in den unteren Respirationstrakt aus. Zellzerstörungen sind in den Flimmerepithelien und den schleimproduzierenden Hautschichten aller Bereiche des Respirationstraktes zu beobachten. Bildet sich eine primäre, interstitielle Lungenentzündung aus, wird das Virus auf die Zellen des Lungenparenchyms übertragen. Man findet starke Anschwellungen der Alveolarwände, deren Epithel durch die Zellzerstörung häufig vollkommen abgetragen ist. Eine solche Anschwellung von Lungengewebe kann durch die Zunahme des Lungengewichtes nachgewiesen werden.

Es existieren sowohl prophylaktische als auch therapeutische Behandlungen. So stehen Impfstoffe gegen Influenza-A und -B Infektionen zur Verfügung. Es handelt sich dabei um abgetötete Viren, die in Hühnereiern und/oder Zellkultur gezüchtet werden. Der Impfschutz hat etwa zwei Wochen nach der Impfung seine volle Wirksamkeit erreicht. Wegen der hohen Variabilität der Influenzaviren müssen die Impfstoffe jedoch jährlich an die aktuell zirkulierenden Virussubtypen bzw. Subtypvarianten angepasst werden.

Weiterhin finden Hemmstoffe der viralen Neuraminidase (Zanamivir, Oseltamivir) Anwendung, die die Loslösung von neu replizierten Viruspartikeln aus der Wirtszelle verhindern. Sie werden bevorzugt kurz nach erfolgter und nachgewiesener Infektion mit Influenzaviren eingesetzt, um die Virusausbreitung in der Frühphase der Infektion einzudämmen (WO 2003/026567 A2).

Die Hemmstoffe der viralen Neuraminidase greifen jedoch nur in die Vermehrung der Viren ein, können aber bereits im Körper befindliche Viren nicht inaktivieren. Neuraminidasehemmer können nur dazu beitragen, dass die Krankheitsdauer geringfügig (im Mittel bei Erwachsenen um einen Tag) verkürzt wird.

Es ist das Ziel der vorliegenden Erfindung, die therapeutische Wirkung derartiger Hemmstoffe der Virusvermehrung deutlich zu erhöhen. Mit der vorliegenden Erfindung sollen verbesserte pharmazeutische Zusammensetzungen zur Behandlung von Infektionen mit Influenzaviren zur Verfügung gestellt werden.

Demgemäß betrifft die vorliegende Erfindung eine Zusammensetzung, umfassend
- ein Peptid, welches aus 7-17 benachbarten Aminosäuren besteht und das Hexamer TX₁EX₂X₃E umfasst, wobei X₁, X₂ und X₃ jede natürliche oder nicht natürliche Aminosäure sein kann, wobei das Peptid keine TNF-Rezeptor-Bindungsaktivität aufweist und zyklisiert ist, und
- ein Hemmstoff der viralen Neuraminidase, insbesondere zur Anwendung zur Verhinderung und Behandlung der Influenza.

Es wurde nun erfindungsgemäß festgestellt, dass die Wirkung von Neuraminidasehemmern überraschend verbessert werden kann, wenn ein Peptid, wie oben definiert, in Kombination mit einem Neuraminidasehemmer zur Behandlung von Influenza-Infektionen eingesetzt wird. Auch ein prophylaktischer Einsatz der erfindungsgemäßen Zusammensetzung ist dadurch angezeigt. Besonders effizient hat sich die vorliegende Erfindung bei der Behandlung von Lungenentzündungen erwiesen, die durch die Influenza-Viren bedingt ist.

Die erfindungsgemäß zu verwendenden Peptide sind beispielsweise aus dem europäischen Patent EP 1 264 599 B1 (oder aus der US 2007/299003 A, WO 94/18325 A1 oder WO 2008/148545 A1) bekannt und wurden im Stand der Technik zur Behandlung von Flüssigkeitsansammlungen (Lungenödemen) und insbesondere zur Reabsorption dieser Flüssigkeitsansammlungen verwendet. Jedoch eignen sich diese Peptide überraschenderweise auch dazu, den entgegengerichteten Flüssigkeitsstrom über das Endothel der Kapillaren in das Epithel der Lunge zu beeinflussen und können daher auch zur Verhinderung und Behandlung der Hyperpermeabilität von Epithelzellen und Endothelzellen verwendet werden (WO 2010/099556 A).

Diese - an sich bekannten - Peptide, die erfindungsgemäß zusammen mit dem Neuraminidasehemmer zum Einsatz kommen, weisen keine TNF-Rezeptor-Bindungsaktivität auf (Hribar et al., Eur. J. Immunol. 1999; Elia et al., AJRCCM 2003; s. auch: Beispielteil unten) und sind zyklisiert. Bevorzugte Varianten dieser Peptide bestehen aus 7-17 benachbarten Aminosäuren und enthalten das Hexamer TPEGAE (SEQ ID Nr. 4).

Während allerdings die WO 2010/099556 A1 wie auch die EP 1 264 599 A1 auf die Behandlung von bestimmten, pathologischen Symptomen in der Lunge abzielen und nicht auf die Verhinderung und Behandlung einer Erkrankung mit einem bestimmten Pathogen, ist die Verwendung gemäß der vorliegenden Erfindung eine komplett andere:
Wie sich nämlich im Zuge der Arbeiten zur vorliegenden Erfindung herausgestellt hat, unterstützen die anspruchsgemäßen Peptide (u.a. gemäß der WO 2010/099556 A1) die Wirkung der Neuraminidasehemmer in synergistischer Weise im erfindungsgemäßen Kombinationspräparat. Die Verwendung gemäß der vorliegenden Erfindung zielt daher direkt auf die Infektion durch Influenza-Viren ab und nicht auf allgemeine Symptome, die durch mehrere verschiedene Pathogene ausgelöst werden können (wie dies beispielsweise in der WO 2010/099556 A1 oder in der EP 1 264 599 A1 der Fall ist).

Es ist zwar aus der Sicht eines Fachmannes auf diesem Gebiet klar, dass Neuraminidasehemmer nur die Vermehrung von Inluenzaviren hemmen können, jedoch bereits lebende Viren dadurch nicht reduziert werden können; es ist allerdings völlig überraschend und durch den Stand der Technik nicht nahegelegt, dass die Kombination aus Neuraminidasehemmern und den anspruchsgemäßen Peptiden zu einer wesentlichen Verbesserung der Bekämpfung der Influenza führt. Im Stand der Technik erhält ein Fachmann keinerlei Anregungen für diese Kombination, im Gegenteil:
In der WO 2003/026567 A2 ist beschrieben, dass die zur Behandlung der Influenza verwendeten Neuraminidasehemmer auch geeignet sind, Influenza-assoziierte bakterielle Infektionen zu verhindern. Ausgehend von diesem Dokument kann ein Fachmann daher überhaupt keine Veranlassung erkennen, zu Neuraminidasehemmern weitere Zusatzstoffe hinzuzufügen, die die antivirale Wirksamkeit bzw. die Effektivität einer Influenza-Behandlung verbessern.

Bei einer besonders bevorzugten Ausführungsform der vorliegenden Erfindung umfasst die erfindungsgemäße Zusammensetzung ein zyklisiertes Peptid, bestehend aus einer Sequenz aufeinanderfolgender Aminosäuren, ausgewählt aus der Gruppe bestehend aus
- QRETPEGAEAKPWY (SEQ ID Nr. 5)
- PKDTPEGAELKPWY (SEQ ID Nr. 6)
- CGQRETPEGAEAKPWYC (SEQ ID Nr. 1) und
- CGPKDTPEGAELKPWYC (SEQ ID Nr. 7)
und Fragmenten von mindestens 7 Aminosäuren davon, welche Fragmente des Hexamer TPEGAE aufweisen.

Vorzugsweise umfasst das Peptid in der Zusammensetzung die Aminosäuresequenz CGQRETPEGAEAKPWYC (SEQ ID Nr. 1) und ist über die C-Reste zyklisiert. Dieses besonders bevorzugte Peptid hat somit folgende Aminosäuresequenz (SEQ ID Nr. 1) (NH2)Cys-Gly-Gln-Arg-Glu-Thr-Pro-Glu-Gly-Ala-Glu-Ala-Lys-Pro-Trp-Tyr-Cys(COOH). Dieses Peptid wird auch als "AP301" bezeichnet.

Die Zyklisierung der erfindungsgemäßen Peptide kann dabei z.B. entweder über eine direkte Zyklisierung über eine Disulfidbrücke zwischen den beiden C-Resten am N- und C-Terminus erreicht werden oder aber indem das Peptid über beide Cysteine an eine Trägersubstanz gekoppelt wird. Dabei werden in den erfindungsgemäßen Peptiden die Cysteinreste vorzugsweise am Anfang und am Ende des Moleküls vorgesehen. Auch andere funktionelle Gruppen, die eine Zyklisierung des Peptids erreichen, können eingesetzt werden, z.B. indem eine Säuregruppe mit einem Amin oder Alkohol zu einem Amid- oder Ester-Ringschluss führt (hierbei können z.B. die Aminosäuren Asparaginsäure und Glutaminsäure mit Serin, Threonin, Tyrosin, Asparagin, Glutamin oder Lysin, vorzugsweise intramolekular zyklisiert werden). Die Zyklisierung des Peptids erfolgt vorzugsweise durch eine Disulfidbrücke zwischen den C-Resten des Peptids (wenn vorhanden).

Weitere bevorzugte erfindungsgemäße Peptide sind daher beispielsweise CGQKETPEGAEAKPWYC (SEQ ID Nr. 8), CGQRETPEGAEARPWYC (SEQ ID Nr. 9), CGQRETPEGAEAKPC (SEQ ID Nr. 10), CQRETPEGAEAKPWYC (SEQ ID Nr. 11) oder CGQRETPEGAEAKFWYC (SEQ ID Nr. 12).

Die Zyklisierung kann aber auch durch Bindung des Peptids an Trägersubstanzen erfolgen. Als derartige Zyklisierungs-Trägersubstanzen kommen alle gängigen pharmazeutisch verwendbaren Substanzen in Frage, die in der Lage sind, z.B. mit den SH-Gruppen der Cysteine (oder mit anderen natürlicherweise vorhandenen oder artifiziell eingeführten chemisch reaktiven Gruppen des Peptids) eine kovalente Bindung einzugehen, wobei gängige Trägerproteine, wie Keyhole limpet hemocyanin (KLH), Tetanus-Toxin etc., sich besonders eignen. Auch können am Träger benachbarte bifunktionelle Reste vorgesehen werden (z.B. Säuregruppe neben Amin- oder Alkoholgruppe). In diesem Zusammenhang ist wichtig, dass mit "Zyklisierung" sowohl der intramolekulare Ringschluss als auch die Einbindung eines Trägers umfasst ist (von dem das gebundene Peptid hervorragt (indem der N- und der C-Terminus des Peptids an den Träger gebunden ist)), wobei das derart zyklisierte Peptid die zyklische Raumstruktur zeigt und entsprechend stabilisiert ist.

Hemmstoffe der viralen Neuraminidase sind bekannt und haben sich bereits bei der Vorbeugung und Behandlung von Influenza bewährt. Derartige virale Neuraminidasehemmer (wie z.B. Zanamivir, Oseltamivir, Laninamivir oder Peramivir) verhindern die Loslösung von neu replizierten Viruspartikeln aus der Wirtszelle. Vor allem kurz nach erfolgter und nachgewiesener Infektion mit Influenzaviren kann damit die Virusausbreitung in der Frühphase der Infektion eingedämmt werden.

Diese Neuraminidasehemmer greifen jedoch nur in die Vermehrung der Viren ein, können aber bereits im Körper befindliche Viren nicht inaktivieren, so dass oft nur die Krankheitsdauer geringfügig (im Mittel bei Erwachsenen um einen Tag) verkürzt werden kann. Vor allem aber können damit in der Regel die Symptome der Influenza, insbesondere eine Lungenentzündung, die meist zu besonderen Komplikationen führt, nicht wirksam genug bekämpft bzw. verhindert werden.

Neuraminidase ist ein wesentliches Enzym für die Reproduktion des Influenza-Virus und sie ist als "molekulare Schere" beschrieben worden, die für die Freisetzung der Viren verantwortlich ist.

Neuraminidasehemmer umfassen Analoga der Sialinsäure, die eine neue Klasse der zweiten Generation antiviraler Mittel repräsentieren, die Wirksamkeit gegen Influenza A und B Viren zeigen. Neuraminidasehemmer gemäß der vorliegenden Erfindung können alle bislang hierfür vorgeschlagenen Verbindungen sein, wie z.B. in der US 2008/0063722 A1 zusammengefasst (auch für bevorzugte pharmazeutische Formulierungen derartiger Substanzen). Diese Stoffe können mindestens eine enzymatische Tätigkeit des Neuraminidase-Proteins eines virulenten Stammes eines Influenza-Virions des Typs A oder des Typs B hemmen. Derartige Stoffe können sowohl zur Prophylaxe als auch zur Behandlung von Influenza verwendet werden; in Kombination mit den oben definierten Peptiden gemäß der vorliegenden Erfindung ist jedoch diese Wirkung signifikant verbessert.

Beispiele von Neuraminidasehemmern, die in der vorliegenden Zusammensetzung zum Einsatz kommen können, sind z.B. in den US-Patenten 5,453,533, 5,763,483, 5,952,375, 5,958,973, 5,512,596, 5,886,213, 5,602,277, 6,410,594, 5,360,817, 5,866,601, 6,340.702, 6,451,766, 6,455,571, 6,593,314, 6,509,359, 6,518,305 und 6,831,096 beschrieben. Substanzen, die bereits am Menschen eingesetzt werden (oder zumindest in der klinischen Erprobung sind) und demgemäß besonders bevorzugt sind, sind beispielsweise CS-8958 (RI 18958; US 2008/0063722 A1), Zanamivir (GG167, RELEN-ZA^{®}), Peramivir (RWJ-270201, BCX-1812), Oseltamivir-Phosphat (Ro64-0796, GS4104), Oseltamivir-Carboxylat (Ro64-0802, GS4071) oder Oseltamivir (GS4104, TAMIFLU^{®}). Selbstverständlich werden erfindungsgemäß die Neuraminidasehemmer in allen wirksamen chemischen Formen umfasst, also als Salze, racemische, optisch reine und/oder salzlose Formen (auch in Form des z.B. Enantiomers oder Diastereomers).

Vorzugsweise ist der Hemmstoff der viralen Neuraminidase Zanamivir oder Oseltamivir; diese Stoffe sind bevorzugt, weil sie bereits besonders erfolgreich zur Behandlung am Menschen eingesetzt werden.

Bevorzugter Weise umfasst die erfindungsgemäße Zusammensetzung einen pharmazeutisch akzeptablen Träger und ist als pharmazeutische Zusammensetzung hergerichtet, die zur Verabreichung am Menschen geeignet ist.

Der Ausdruck "eine pharmazeutische Zusammensetzung" bezieht sich auf jede Zusammensetzung, die einen Neuraminidasehemmer und ein Peptid, wie oben definiert, umfasst (natürlich auch geeignete (i.e. nicht miteinander negativ interferierende) Gemische derartiger Substanzen), welche die hierin beschriebenen Zustände verhindert, verbessert oder heilt. Insbesondere bezieht sich der Ausdruck "eine pharmazeutische Zusammensetzung" auf eine Zusammensetzung, die einen Neuraminidasehemmer und ein Peptid, wie oben beschrieben, und einen pharmazeutisch akzeptablen Träger oder Exzipienten (beide Ausdrücke können austauschbar verwendet werden) aufweist. Geeignete Beispiele von Trägern oder Exzipienten, die dem Fachmann bekannt sind, sind Wasser, Kochsalzlösung, Natriumphosphat, Natriumacetat, Natriumcarbonat, Zitrat, Glycin, Glycylglycin, Histidin, Lysin, Arginin, TRIS und Natriumcitrat oder Mischungen davon. Natürlich können auch Ringer-Lösung, Dextrose-Lösung oder Lösungen nicht reduzierbarer Zucker eingesetzt werden; demgemäß eignen sich auch Mannit, Trehalose, Saccharose, Sorbit, Fruchtzucker, Maltose, Lactose oder Dextran, Hank-Lösung, fixierte Öle, Ethyloleat, 5% Dextrose in Kochsalzlösung, Substanzen, die die Isotonie und die chemische Stabilität verbessern, Puffer und Konservierungsmittel als derartige Träger. Andere geeignete Träger inkludieren jeden Träger, der nicht selbst die Produktion von Antikörpern, die für das die Zusammensetzung erhaltende Individuum schädlich sind, induziert, wie Proteine, Polysaccharide, Polymilchsäuren, Polyglykolsäuren, polymere Aminosäuren und Aminosäure-Copolymere. Bei der Formulierung der erfindungsgemäßen Zusammensetzung sind natürlich die einschlägigen Richtlinien (z.B. die (europäische oder US) Pharmakopöe) zu beachten. Dabei kann das in der erfindungsgemäßen Zusammensetzung vorgesehene Peptid auch durch direkte kovalente Bindung an diese Träger zyklisiert werden.

Die erfindungsgemäße pharmazeutische Zusammensetzung kann (als Medikament) mit jedem im Wissen des Fachmanns liegenden geeigneten Verfahren verabreicht werden, insbesondere ist bevorzugt, das erfindungsgemäß zu verwendende Peptid bzw. die erfindungsgemäße Zusammensetzung in die Lunge zu verabreichen. Obgleich Influenza auch bei Tieren eine gewisse Rolle spielt und die erfindungsgemäße Zusammensetzung natürlich auch zur Vorbeugung und Prophylaxe bei Tieren eingesetzt werden kann, liegt doch der Schwerpunkt der vorliegenden Erfindung auf der Vorbeugung und Behandlung am Menschen, also von Personen, die mit Influenza bereits infiziert worden sind oder die Gefahr laufen, sich mit diesem Virus zu infizieren (insbesondere bei Influenza-Epidemien oder -Pandemien). Der bevorzugte Verabreichungsweg ist Inhalation (durch Aerosole) aber auch die intravenöse Gabe, Instillation, orale Gabe oder Kombinationen daraus. Bei inhalativer, parenteraler oder oraler Verabreichung wird das Medikament dieser Erfindung in Dosiseinheitsform, wie als Lösung, Suspension oder Emulsion, in Verbindung mit den oben definierten pharmazeutisch akzeptablen Exzipienten formuliert. Die Dosierung und Verabreichungsart kann jedoch natürlich auch im Einzelfall vom jeweiligen Individuum abhängen.

Dabei wird die jeweils erforderliche wirksame Menge an das Individuum, das die Verabreichung benötigt, verabreicht. Dabei ist die "wirksame Menge" als eine Menge zu verstehen, die wirksam genug ist, um den beabsichtigten therapeutischen oder prophylaktischen Effekt zu erzielen, also z.B. eine weitere Ausbreitung der Erkrankung zu verhindern oder wirksam zu behandeln. Dabei wird meist von einem durchschnittlichen Patienten ausgegangen, jedoch können die tatsächlichen wirkungsvollen Mengen der Komponenten in der Zusammensetzung so formuliert werden, dass die Art der Verabreichung und das Alter, Gewicht, Zustand des Patienten und Grad und Fortschritt der Erkrankung berücksichtigt werden (z.B. mittels eines passenden, herkömmlichen pharmakologischen Protokolls).

Vorzugsweise ist daher der pharmazeutisch akzeptable Träger in der erfindungsgemäßen Zusammensetzung ausgewählt aus Wasser (besonders bevorzugt: Wasser für Injektion), Kochsalz, Natriumphosphat, Natriumacetat, Natriumcarbonat, Zitrat, Glycin, Glycylglycin, Histidin, Lysin, Arginin, TRIS, Natriumcitrat, Ringer-Lösung, Dextrose, Mannit, Trehalose, Saccharose, Sorbit, Fruchtzucker, Maltose, Lactose oder Dextran, Hank-Lösung, fixierte Öle, Ethyloleat, Substanzen, die die Isotonie und die chemische Stabilität verbessern, Konservierungsmittel, pharmazeutisch akzeptable Proteine, Polysaccharide, Polymilchsäuren, Polyglykolsäuren, polymere Aminosäuren und Aminosäure-Copolymere.

Auch kann die erfindungsgemäße Zusammensetzung bereitgestellt werden, indem die beiden Wirkkomponenten, also der Neuraminidasehemmer und das Peptid, wie oben beschrieben, in räumlich getrennter Form vorgesehen werden, also als Set, welches zumindest einen Neuraminidasehemmer und ein Peptid (jeweils in voneinander getrennten Behältnissen) umfasst. Demgemäß betrifft die vorliegende Erfindung auch ein Set, welches zumindest einen Neuraminidasehemmer und ein Peptid (jeweils in voneinander getrennten Behältnissen) umfasst. Die dadurch ermöglichte (räumlich, aber auch zeitlich) getrennte Gabe von Peptid und dem Hemmstoff ist vor allem dann bevorzugt, wenn verschiedene Verabreichungswege der beiden erfindungsgemäßen Wirkkomponenten für den betroffenen Patienten gewünscht sind. Beispielsweise wird Oseltamivir zumeist oral gegeben, das erfindungsgemäß zu verwendende Peptid aber zumeist inhalativ. Es ist oft aber auch die gleichzeitige Gabe erwünscht, beispielsweise wird Zanamivir ebenfalls inhalativ verabreicht. Natürlich kann auch angezeigt sein, dass ein oraler Hemmstoff mittels Inhalation systemisch zur Verfügung gestellt wird. In manchen Fällen kann das erfindungsgemäß zu verwendende Peptid nur schwer mit dem Neuraminidasehemmer zusammengemischt werden, z.B. wenn der Hemmstoff i.v. oder oral gegeben wird und das Peptid inhalativ. In vielen Fällen kann aber sowohl Hemmstoff und Peptid zusammen inhalativ verabreicht werden, wobei der Hemmstoff dann direkt in die Lunge und gegebenenfalls durch diese ins Blut gelangt (Influenza-Viren sind zuerst im Lungengewebe präsent).

In einem derartigen erfindungsgemäßen Set, umfassend getrennte Neuraminidase- und Peptid-Komponenten, können natürlich ebenfalls die hierin beschriebenen Merkmale und bevorzugten Ausführungsformen für die gemischte Zusammensetzung in allen für den Fachmann hieraus vorstellbaren Kombinationen vorgesehen werden.

Das erfindungsgemäße Medikament kann beispielsweise so verabreicht werden, dass das Peptid der vorliegenden Erfindung in einer Dosis von zwischen 1 µg/kg und 10 mg/kg, mehr bevorzugt zwischen 10 µg/kg und 5 mg/kg, am meisten bevorzugt zwischen 0,1 und 2 mg/kg, gegeben wird. Vorzugsweise wird es als Bolus-Dosis gegeben. Es kann aber auch eine kontinuierliche Inhalation oder Infusion oder eine Verabreichung mittels wiederholter Gabe verwendet werden.

Besonders bevorzugte erfindungsgemäße Zusammensetzungen enthalten - unabhängig voneinander -
- das Peptid in einer Menge von 1 µg bis 10 g, vorzugsweise von 10 µg bis 1 g, insbesondere von 1 mg bis 100 mg, und
- den Hemmstoff der viralen Neuraminidase in einer Menge von 1 µg bis 10 g, vorzugsweise von 100 µg bis 1 g, insbesondere von 1 mg bis 200 mg.

Besonders bevorzugte erfindungsgemäße Zusammensetzungen in flüssiger Form enthalten - unabhängig voneinander -
- das Peptid in einer Menge von 1 µg bis 10 g, vorzugsweise von 10 µg bis 1 g, insbesondere von 1 mg bis 100 mg, und
- den Hemmstoff der viralen Neuraminidase in einer Menge von 1 µg bis 10 g, vorzugsweise von 100 µg bis 1 g, insbesondere von 1 mg bis 200 mg,
und liegen in einem Volumen von 0,5 bis 10 ml, insbesondere in einem Volumen von 1 bis 5 ml, vor.

Die erfindungsgemäße Zusammensetzung kann vorzugsweise auch in Trockenform mittels Pulverinhalator verabreicht werden. Beispiele für derartige Pulverinhalatoren, die für die vorliegende Erfindung eingesetzt werden können, sind in den US-Patenten 4.995.385 und 4.069.819 beschrieben; bereits etablierte Produkte sind SPINHALER^{®}, ROTAHALER^{®}, FLOWCAPS^{®}, INHALATOR^{®}, DISKHALER^{®} und AEROLIZER^{®}.

Die erfindungsgemäße Zusammensetzung kann vorzugsweise auch als Aerosol mittels Flüssigkeitsvernebler verabreicht werden. Beispiele für derartige Flüssigkeitsvernebler sind etablierte Produkte wie Aeroneb^{®} und Pari^{®}.

Gemäß einer bevorzugten Ausführungsform ist die erfindungsgemäße Zusammensetzung dadurch gekennzeichnet, dass das Peptid und/oder der Hemmstoff der viralen Neuraminidase in einer vernebelbaren Pulverformulierung oder in einer vernebelbaren Flüssigformulierung vorliegen.

Die erfindungsgemäße Zusammensetzung wird vorzugsweise zur Behandlung und Prophylaxe von Infektionen mit dem Influenza-Virus Typ A und Typ B, insbesondere Typ A, eingesetzt. Jedoch ist die Zusammensetzung prinzipiell auch geeignet, Infektionen mit jedem möglichen Stamm des Influenza-Virus vorzubeugen oder zu behandeln, der diese Krankheit in einem Tier oder in einem Menschen verursachen kann. Entsprechende Datenbanken mit Informationen zu den verschiedenen Influenza-Typen sind den Fachleuten auf diesem Gebiet vertraut, insbesondere sind viele isolierte Stämme vom Typ A beschrieben oder sogar sequenziert.

Die Erfindung wird anhand der nachfolgenden Beispiele und der Zeichnungsfiguren, auf die sie aber selbstverständlich nicht beschränkt ist, näher erläutert.

Es zeigen:
Fig. 1: das relative Lungengewicht von C57BL/6-Mäusen an den Tagen 3, 5, 7 und 9 nach Infektion mit Influenza A bzw. (als Kontrolle) ohne Infektion;
Fig. 2: das relative Lungengewicht von C57BL/6-Mäusen an den Tagen 5, 7 und 9 nach Infektion mit Influenza A und Behandlung mit Oseltamivir, Behandlung mit der erfindungsgemäßen Zusammensetzung und ohne Behandlung (mit PBS; als Kontrolle);
Fig. 3: das relative Lungengewicht von C57BL/6-Mäusen an den Tagen 7 und 9 nach Infektion mit Influenza A und Behandlung mit Zanamivir, Behandlung mit der erfindungsgemäßen Zusammensetzung und ohne Behandlung (mit PBS; als Kontrolle).

### Beispiele

Mit den vorliegenden Beispielen wird in einem anerkannten experimentellen Mausmodell gezeigt, dass die erfindungsgemäße Zielstellung erreicht wurde, indem an mit Influenza Virus infizierten Mäusen sowohl ein Neuraminidasehemmstoff als auch eine Kombination des Neuraminidasehemmstoffes und des synthetischen Peptids AP301 (SEQ. ID. Nr.1) verabreicht wurde.

### Beispiel 1

Infektion mit Influenza Virus bewirkt die Herausbildung einer Lungenentzündung.

Labormäuse (Stamm C57BL/6, 8 Wochen alt) wurden per nasal mit dem Influenzastamm A (PR8/34) und einer Dosierung von 150 PFU infiziert. Am 3., am 5., am 7. und am 9. Tag nach der Infektion wurden jeweils von acht Mäusen die Lungen entnommen und das relative Lungengewicht als Maß für die Lungenentzündung bestimmt.

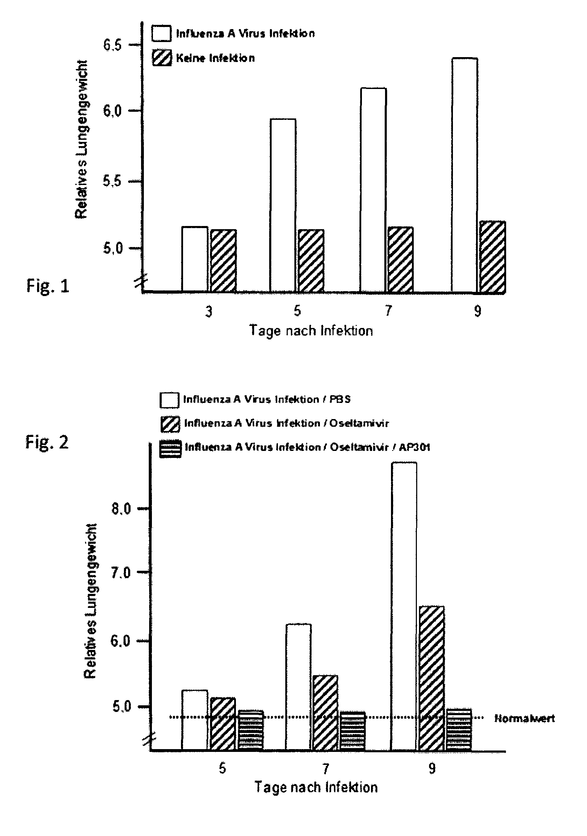

Die Untersuchung ergab, dass mit zunehmender Dauer nach Infektion der Mäuse mit dem Influenza Virus das Lungengewicht im Vergleich mit Kontrolllungen stark zunahm. Die Ergebnisse sind in Fig. 1 graphisch dargestellt.

### Beispiel 2

Behandlung der Lungenentzündung durch Verabreichung von Neuraminidasehemmer oder Verabreichung einer Kombination aus Neuraminidasehemmer und Peptid AP301.

Labormäuse (Stamm C57BL/6, 8 Wochen alt) wurden per nasal mit dem Influenzastamm A (PR8/34) und einer Dosierung von 150 PFU infiziert. Anschließend erhielten die Versuchstiere jeweils orale Gaben von 10 mg/kg Oseltamivir (Neuraminidasehemmstoff) und intratracheale Gaben von 10 µg/Testtier Peptid AP301. Die Behandlung wurde am 2. und 4. Testtag wiederholt.

Am 5., am 7. und am 9. Tag nach der Infektion wurden jeweils von 30 Mäusen die Lungen entnommen und das relative Lungengewicht als Maß für die Lungenentzündung bestimmt. Die Ergebnisse sind in Fig. 2 graphisch dargestellt.

Die Untersuchung ergab, dass der Neuraminidasehemmstoff nur eine moderate Wirkung zur Senkung der Lungenentzündung, gemessen durch das Lungengewicht, ausübte. Wurde jedoch den mit Influenza-Viren infizierten Mäusen zusätzlich zum Neuraminidasehemmstoff das Peptid AP301 verabreicht, konnte die Lungenentzündung wesentlich stärker reduziert werden.

### Beispiel 3

Ex vivo Beurteilung der pro-inflammatorischen Eigenschaften des AP301 Peptids im menschlichen Vollblut.

Eine pharmakologische ex-vivo-Sicherheitsstudie hinsichtlich des AP301 Peptids im menschlichen Vollblut wurde durchgeführt, um festzustellen, ob das AP301-Peptid zur Freisetzung des pro-inflammatorischen Markers Interleukin-6 (IL-6) aus frischem Vollblut führt (d.h. ob APN 301 TNF-spezifische entzündliche Aktivität (i.e. TNF-Rezeptor-Bindungsaktivität) zeigt oder nicht). In dieser Studie ist frisches Vollblut verwendet worden, hierbei handelt es sich um ein anerkanntes Vorhersagemodell für die Beurteilung der Entzündungsreaktion in vivo.

### Zusammenfassung der Methodik

Es war das Ziel dieser Studie, die proinflammatorische Signal-Kapazität des AP301 Peptids zu bestimmen. Dabei wurden Vollblut-Kulturen verwendet und die Sekretion von Interleukin-6 (IL-6), ein sehr sensitiver Marker für proinflammatorische Stimulation, mittels ELISA quantifiziert.

### Testsystem

| | |
|---|---|
| Testsystem | 25 ml frisch entnommenes heparinisiertes Blut von 5 gesunden Probanden (GP) wurde in den Tests verwendet. |
| Prüfgegenstand | |
| Identifikation: | AP301 Peptid (Dosis: 1 ng/ml bis 10 µg/ml; einmalige Verabreichung in Lösung) |
| Beschreibung: | Weißes Pulver, Reinheit 96% |

### Vollblut-Kulturen

Vollblut (VB)-Kulturen wurden durch Pipettieren von 1 ml VB in Vertiefungen von 24-Näpfchen-Platten durchgeführt. In jedem Experiment wurden unstimulierte und stimulierte KontrollKulturen inkludiert.

Wenn möglich wurden die zu untersuchenden Substanzen und Stimulanzien immer in gleichem Volumen in jedem Näpfchen in einem gegebenen Experiment verwendet, welches nicht größer als 10% des Gesamtvolumens in einem Näpfchen ist. Unstimulierte Kontrollen erfolgten mit PBS. Volumeneinstellung und Verdünnungen für verschiedene Behandlungen wurden ebenfalls mit PBS durchgeführt.

Der Inhalt jedes Näpfchens wurde gemischt und die Platten bei 37°C und 5% CO₂ für 24 Stunden inkubiert. Nach der Inkubation wurde der Inhalt jedes Näpfchens in ein frisches 1,5 ml Mikroröhrchen überführt und bei 8000 bis 9000 x g für 15 Minuten zentrifugiert. Der Überstand jeder Probe wurde einzeln auf zwei 1,5 ml Reaktionsgefäße aufgeteilt und bei -20°C bis zum Gebrauch gelagert.

### Nachweis von Interleukin-6

Interleukin-6 wurde mittels eines spezifischen ELISA quantifiziert (Human IL-6 ELISA-Set, BD Biosciences, Cat. Nr. 555220) unter Einsatz eines Anti-Human-IL-6 Antikörpers als Fänger-Antikörper, eines biotinylierten anti-human IL-6 Detektionsantikörpers, Avidin-Meerrettichperoxidase-Konjugat als Enzym-Reagenz und rekombinantem IL-6 als Standard. Absorptionsmessung bei 450 nm wurde mit dem Packard FusionReader durchgeführt.

### Datenanalyse

Die Ergebnisse jeder Platte wurden gespeichert und mit der FusionDataAnalysis Software ausgewertet.

### Zusammenfassung der Ergebnisse der Studie

Es war das Ziel dieser Studie, die pro-inflammatorische Signalisierungs-Kapazität des AP301 Peptids zu bestimmen. Vollblut-Kulturen wurden verwendet und die Sekretion von IL-6, einem sehr sensitiven Marker für entzündliche Pro-Stimulation, wurde mittels ELISA quantifiziert.

Vollblutproben von fünf gesunden Probanden wurden entweder unstimuliert belassen (negative Kontrolle), stimuliert mit hohen und niedrigen Dosen von LPS (positive Kontrollen) oder mit dem Peptid in neun halblogarithmischen Verdünnungen von 10 µg/ml bis 1 ng/ml inkubiert. Die Ergebnisse sind in der nachfolgenden Tabelle dargestellt:

**Tabelle: Freisetzung von Interleukin-6 aus frischem Vollblut bei Zugabe von Peptid AP301 und LPS**

| | AP301 Peptid | Positive Kontrolle (LPS) |
|---|---|---|
| Konzentration | Konzentration von IL-6 (pg/ml, n = 5) | |
| 0 (Negativkontrolle) | weniger als 0,5 | weniger als 0,5 |
| 10 mg/ml | weniger als 0,5 | 195,640 |
| 1 mg/ml | weniger als 0,5 | 108,370 |
| 3 ng/ml | weniger als 0,5 | 34,867 |
| 1 ng/ml | weniger als 0,5 | nicht bestimmt |

Die Ergebnisse zeigen deutlich, dass das AP301-Peptid keine nachweisbare Menge von IL-6-Sekretion bei keinem der getesteten Konzentrationen induzierte. Die positiven Kontrollen (LPS) führten zu einer starken Induktion der IL-6-Sekretion.

### Diskussion

Die Versuche wurden durchgeführt, um festzustellen, ob das AP301-Peptid die Induktion einer pro-inflammatorischen Kaskade vermittelt. Readout-Parameter war die induzierte Sekretion von IL-6 in Vollblut-Kulturen aus fünf gesunden Spendern. Die Ergebnisse zeigten deutlich, dass das AP301-Peptid kein nachweisbares Niveau von IL-6 in den Spenderkulturen induzierte. Damit ist bewiesen, dass das AP301-Peptid keine pro-inflammatorische Antwort in dem gewählten ex vivo-Modell induziert und somit keine TNF-Rezeptor-Bindungsaktivität aufweist.

### Beispiel 4

Behandlung der Lungenentzündung durch Verabreichung von Neuraminidasehemmer (Zanamivir) oder Verabreichung einer Kombination aus Neuraminidasehemmer (Zanamivir) und Peptid AP301.

Labormäuse (Stamm C57BL/6, 8 Wochen alt) wurden per nasal mit dem Influenzastamm A (PR8/34) und einer Dosierung von 150 PFU infiziert. Anschließend erhielten die Versuchstiere jeweils nasale Gaben von 1,5 mg/kg Zanamivir (Neuraminidasehemmstoff) und intratracheale Gaben von 50 µg/Testtier Peptid AP301. Die Behandlung wurde am 2. und 4. Testtag wiederholt.

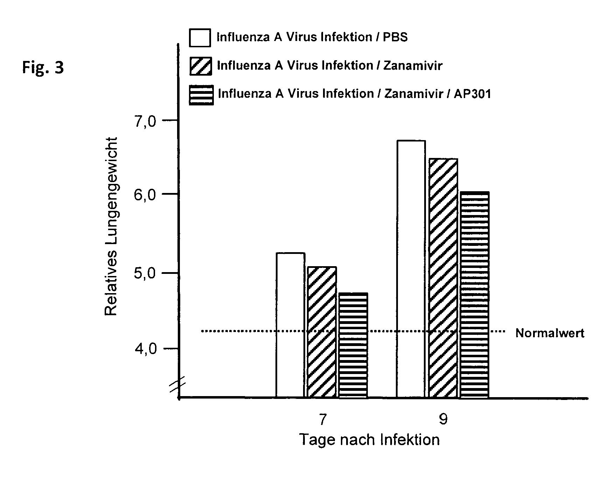

Am 7. und am 9. Tag nach der Infektion wurden jeweils von 20 Mäusen die Lungen entnommen und das relative Lungengewicht als Maß für die Lungenentzündung bestimmt. Die Ergebnisse sind in Fig. 3 graphisch dargestellt.

Die Untersuchung ergab, dass der Neuraminidasehemmstoff (Zanamivir) nur eine moderate Wirkung zur Senkung der Lungenentzündung, gemessen durch das Lungengewicht, ausübte. Wurde jedoch den mit Influenza-Viren infizierten Mäusen zusätzlich zum Neuraminidasehemmstoff (Zanamivir) das Peptid AP301 verabreicht, konnte die Lungenentzündung wesentlich stärker reduziert werden.

### Zusammenfassung

Die erfindungsgemäßen Peptide weisen in Kombination mit einem Neuraminidasehemmstoff eine synergistische Wirkung bei der Behandlung von Influenza-Infektionen auf.

In der WO 2010/099556 A1 wird, wie erwähnt, eine Behandlung von verschiedenen pulmonären Symptomen belegt, die auf die Hyperpermeabilität von Epithelzellen und Endothelzellen bei derartigen Lungenerkrankungen abzielen. Gemäß der WO 2010/099556 A1 hat sich gezeigt, dass die beanspruchten Peptide zur Verhinderung und Behandlung dieser Symptome exzellent geeignet sind. Obgleich gemäß der WO 2010/099556 A1 eine Hyperpermeabilität der Epithelzellen und Endothelzellen auch bei einer Infektionen mit Influenza-Viren (im Rahmen einer mit dieser Infektion auftretenden Pneumonie) behandelt werden kann, wird dadurch die vorliegende Erfindung selbstverständlich nicht nahegelegt. Die Möglichkeit der generellen Behandlung einer Influenza mit einem Kombinationspräparat, enthaltend diese Peptide, stellt eine völlig neue und erfinderische Lehre gegenüber der Eignung der Peptide zur Behandlung eines - fakultativ - mit Influenza auftretenden Symptoms dar.

Der grundlegende Unterschied ergibt sich auch durch einen Blick in die detaillierten Experimente gemäß der WO 2010/099556 A1: In den Beispielen der WO 2010/099556 A1 wird experimentell dargestellt, dass die Peptide im Lungengewebe:
i) Den Gehalt an reaktivem Sauerstoff beeinflussen,
ii) die Wirkung von bakteriellen Gram-positiven Toxinen "Listeriolysin" und "Pneumolysin" auf eine Hyperpermeabilität beeinflussen, u.a. durch Regulation des Gehalts an phosphorylierter Myosin Light Chain, Infiltration von Leukozyten, aktivierter Protein Kinase C,
iii) nach Influenza-Infektion das Körpergewicht beeinflussen,
iv) nach Influenza-Infektion die Körpertemperatur beeinflusse,
v) nach Influenza-Infektion die Überlebensrate der Versuchstiere beinflussen.

Jedoch gibt es in der gesamten WO 2010/099556 A1 keine experimentellen Hinweise darauf, dass die Infektion von Versuchstieren mit Influenza-Virus zu einer Veränderung des relativen Lungengewichts führt, und dass ein solcher Prozess durch Verabreichung des Peptides aufgehalten und behandelt werden kann. Es wird überhaupt erst mit der vorliegenden Erfindung gezeigt, dass die Infektion von Lungen von Testtieren mit Influenza-Virus zu einer wesentlichen Erhöhung des relativen Lungengewichts führt.

Die Lunge stellt als Organ eines der wichtigsten Organe überhaupt dar. Kommt es zu einer Erhöhung des relativen Lungengewichtes, ist die mit einer Schädigung der Lungenfunktion verbunden, und dies kann durch kein anderes Organ kompensiert werden. Es ist eine Eigenschaft der gesunden Lunge, möglichst viel luftgefüllte Räume (Alveolen) zu enthalten. Bei Zunahme des relativen Lungengewichts ist stark davon auszugehen, dass der Anteil an luftgefüllten Alveolen stark abnimmt und somit die Funktion der Lunge eingeschränkt wird. Daher stellt das relative Lungengewicht einen wesentlichen Faktor zur Behandlung der Influenza dar. Die mit den in der vorliegenden Anmeldung enthaltenen Experimenten gezeigten Resultate belegen daher eindrucksvoll den synergistischen Effekt des erfindungsgemäßen Kombinationspräparates anhand eines äußerst kritischen und relevanten Parameters.

Von den Neuraminidase-Inhibitoren ist bisher bekannt, dass diese die Vermehrung von Influenza-Virus unterdrücken können. Neuraminidase-Inhibitoren führen nicht zu einer Verringerung von lebenden Influenza-Viren. Bislang konnte im Stand der Technik nicht gezeigt werden, dass Influenza-Virus zu einer Erhöhung des relativen Lungengewichts führt. Erst mit der vorliegenden Erfindung wird überraschend gezeigt, dass die Gabe eines Neuraminidase Inhibitors nach einer Influenza-Infektion die relative Gewichtszunahme des Lungengewichts verringert. Zusätzlich konnte mit der vorliegenden Erfindung erstmalig gezeigt werden, dass eine zeitgleiche Behandlung von mit Influenza-Virus infizierten Testtieren mit einer Kombination aus Neuraminidase-Inhibitor und einem Peptid gemäß der WO 2010/099556 A1 zu einem deutlichen und nicht vorhersehbaren synergistischen Effekt auf das relative Lungengewicht führt. Während Neuraminidase-Inhibitoren die Vermehrung von Influenza-Virus hemmen soll, jedoch die bereits lebenden Viren nicht reduziert werden, führt offenbar die synergistische Kombination aus Neuraminidase-Inhibitor und einem erfindungsgemäßen Peptid zu einer wesentlichen Verbesserung der Influenza-Behandlung. Jedes der einzeln verabreichten Arzneimittel (Neuraminidase-Inhibitor und die anspruchsgemäßen Peptide) für sich allein führt nicht zu der mit der vorliegenden Erfindung gezeigten Wirkung.

Die vorliegende Erfindung kann daher durch die in der WO 2010/099556 A1 geoffenbarten Ergebnisse in keiner Weise nahegelegt werden.

### Zusammenfassung der Sequenzen:

SEQ ID Nr. 1 CGQRETPEGAEAKPWYC
SEQ ID Nr. 2 KSPGGQRETPEGAEAKPWYE
SEQ ID Nr. 3 CGQREAPAGAAAKPWYC
SEQ ID Nr. 4 TPEGAE
SEQ ID Nr. 5 QRETPEGAEAKPWY
SEQ ID Nr. 6 PKDTPEGAELKPWY
SEQ ID Nr. 7 CGPKDTPEGAELKPWYC
SEQ ID Nr. 8 CGQKETPEGAEAKPWYC
SEQ ID Nr. 9 CGQRETPEGAEARPWYC
SEQ ID Nr. 10 CGQRETPEGAEAKPC
SEQ ID Nr. 11 CQRETPEGAEAKPWYC
SEQ ID Nr. 12 CGQRETPEGAEAKFWYC

## Patentansprüche

1. Zusammensetzung, umfassend
- ein Peptid, welches aus 7-17 benachbarten Aminosäuren besteht und das Hexamer TX₁EX₂X₃E umfasst, wobei X₁, X₂ und X₃ jede natürliche oder nicht natürliche Aminosäure sein kann, wobei das Peptid keine TNF-Rezeptor-Bindungsaktivität aufweist und zyklisiert ist, und
- ein Hemmstoff der viralen Neuraminidase.

2. Zusammensetzung nach Anspruch 1, wobei das Peptid aus 7-17 benachbarten Aminosäuren besteht und das Hexamer TPEGAE umfasst.

3. Zusammensetzung nach Anspruch 1 oder 2, wobei das zyklisierte Peptid aus einer Sequenz aufeinander folgender Aminosäuren, ausgewählt aus der Gruppe bestehend aus QRETPEGAEAKPWY, PKDTPEGAELKPWY, CGQRETPEGAEAKPWYC, CGPKDTPEGAELKPWYC und Fragmenten von mindestens 7 Aminosäuren davon, welche Fragmente das Hexamer TPEGAE aufweisen, besteht.

4. Zusammensetzung nach einem der Ansprüche 1 bis 3, **dadurch gekennzeichnet, dass** das Peptid die Aminosäuresequenz CGQRETPEGAEAKPWYC umfasst und über die C-Reste zyklisiert ist.

5. Zusammensetzung nach einem der Ansprüche 1 bis 4, **dadurch gekennzeichnet, dass** das Peptid durch eine Disulfidbrücke zwischen den C-Resten zyklisiert ist.

6. Zusammensetzung nach einem der Ansprüche 1 bis 5, **dadurch gekennzeichnet, dass** der Hemmstoff der viralen Neuraminidase Zanamivir oder Oseltamivir ist.

7. Zusammensetzung nach einem der Ansprüche 1 bis 6, **dadurch gekennzeichnet, dass** sie einen pharmazeutisch akzeptablen Träger umfasst und als pharmazeutische Zusammensetzung hergerichtet ist, die zur Verabreichung am Menschen geeignet ist.

8. Zusammensetzung nach einem der Ansprüche 1 bis 7, **dadurch gekennzeichnet, dass** sie einen pharmazeutisch akzeptablen Träger umfasst, der ausgewählt ist aus Wasser, insbesondere Wasser für Injektion, Kochsalz, Natriumphosphat, Natriumacetat, Natriumcarbonat, Zitrat, Glycin, Glycylglycin, Histidin, Lysin, Arginin, TRIS, Natriumcitrat, Ringer-Lösung, Dextrose, Mannit, Trehalose, Saccharose, Sorbit, Fruchtzucker, Maltose, Lactose oder Dextran, Hank-Lösung, fixierte Öle, Ethyloleat, Substanzen, die die Isotonie und die chemische Stabilität verbessern, Konservierungsmittel, pharmazeutisch akzeptable Proteine, Polysaccharide, Polymilchsäuren, Polyglykolsäuren, polymere Aminosäuren und Aminosäure-Copolymere.

9. Zusammensetzung nach einem der Ansprüche 1 bis 8, **dadurch gekennzeichnet, dass** sie - unabhängig voneinander -
- das Peptid in einer Menge von 1 µg bis 10 g, vorzugsweise von 10 µg bis 1 g, insbesondere von 1 mg bis 100 mg, und
- den Hemmstoff der viralen Neuraminidase in einer Menge von 1 µg bis 10 g, vorzugsweise von 100 µg bis 1 g, insbesondere von 1 mg bis 200 mg,
umfasst.

10. Zusammensetzung nach einem der Ansprüche 1 bis 9, **dadurch gekennzeichnet, dass** sie in flüssiger Form vorliegt und - unabhängig voneinander -
- das Peptid in einer Menge von 1 µg bis 10 g, vorzugsweise von 10 µg bis 1 g, insbesondere von 1 mg bis 100 mg, und
- den Hemmstoff der viralen Neuraminidase in einer Menge von 1 µg bis 10 g, vorzugsweise von 100 µg bis 1 g, insbesondere von 1 mg bis 200 mg,
umfasst und in einem Volumen von 0,5 bis 10 ml, insbesondere in einem Volumen von 1 bis 5 ml, vorliegt.

11. Zusammensetzung nach einem der Ansprüche 1 bis 10, **dadurch gekennzeichnet, dass** das Peptid und/oder der Hemmstoff der viralen Neuraminidase in einer vernebelbaren Pulverformulierung oder in einer vernebelbaren Flüssigformulierung vorliegen.

12. Set, umfassend die folgenden, jeweils in voneinander getrennten Behältnissen vorgesehenen Komponenten:
- ein Peptid, welches aus 7-17 benachbarten Aminosäuren besteht und das Hexamer TX₁EX₂X₃E umfasst, wobei X₁, X₂ und X₃ jede natürliche oder nicht natürliche Aminosäure sein kann, wobei das Peptid keine TNF-Rezeptor-Bindungsaktivität aufweist und zyklisiert ist, und
- ein Hemmstoff der viralen Neuraminidase.

13. Set nach Anspruch 12, **dadurch gekennzeichnet, dass** die jeweils in voneinander getrennten Behältnissen vorgesehenen Komponenten wie in einem der Ansprüche 1 bis 11 definiert sind.

14. Zusammensetzung, umfassend
- ein Peptid, welches aus 7-17 benachbarten Aminosäuren besteht und das Hexamer TX₁EX₂X₃E umfasst, wobei X₁, X₂ und X₃ jede natürliche oder nicht natürliche Aminosäure sein kann, wobei das Peptid keine TNF-Rezeptor-Bindungsaktivität aufweist und zyklisiert ist, und
- ein Hemmstoff der viralen Neuraminidase
zur Anwendung zur Verhinderung und Behandlung der Influenza.

15. Zusammensetzung, umfassend
- ein Peptid, welches aus 7-17 benachbarten Aminosäuren besteht und das Hexamer TX₁EX₂X₃E umfasst, wobei X₁, X₂ und X₃ jede natürliche oder nicht natürliche Aminosäure sein kann, wobei das Peptid keine TNF-Rezeptor-Bindungsaktivität aufweist und zyklisiert ist, und
- ein Hemmstoff der viralen Neuraminidase
zur Anwendung bei einer Lungenentzündung, die durch die Influenza verursacht ist.

16. Set, umfassend die folgenden jeweils in voneinander getrennten Behältnissen vorgesehenen Komponenten:
- ein Peptid, welches aus 7-17 benachbarten Aminosäuren besteht und das Hexamer TX₁EX₂X₃E umfasst, wobei X₁, X₂ und X₃ jede natürliche oder nicht natürliche Aminosäure sein kann, wobei das Peptid keine TNF-Rezeptor-Bindungsaktivität aufweist und zyklisiert ist, und
- ein Hemmstoff der viralen Neuraminidase
zur Anwendung zur Verhinderung und Behandlung der Influenza.

## Claims

1. A composition, comprising
- a peptide, which consists of 7-17 adjacent amino acids and comprises the hexamer TX₁EX₂X₃E, wherein X₁, X₂ and X₃ can be any natural or non-natural amino acid, wherein the peptide has no TNF receptor binding activity and is cyclized, and
- an inhibitor of viral neuraminidase.

2. The composition according to claim 1, wherein the peptide consists of 7-17 adjacent amino acids and comprises the hexamer TPEGAE.

3. The composition according to claim 1 or 2, wherein the cyclized peptide consists of a sequence of consecutive amino acids, selected from the group consisting of QRETPEGAEAKPWY, PK-DTPEGAEALKPWY, CGQRETPEGAEAKPWYC, CGPKDTPEGAELKPWYC, and fragments of at least 7 amino acids thereof, which fragments include the hexamer TPEGAE.

4. The composition according to any one of claims 1 to 3, **characterized in that** the peptide comprises the amino acid sequence CGQRETPEGAEAKPWYC and is cyclized via the C-residues.

5. The composition according to any one of claims 1 to 4, **characterized in that** the peptide is cyclized via a disulfide bridge between said C-residues.

6. The composition according to any one of claims 1 to 5, **characterized in that** the inhibitor of viral neuraminidase is Zanamivir or Oseltamivir.

7. The composition according to any one of claims 1 to 6, **characterized in that** it comprises a pharmaceutically acceptable carrier and is prepared as a pharmaceutical composition acceptable for administration to humans.

8. The composition according to any one of claims 1 to 7, **characterized in that** it comprises a pharmaceutically acceptable carrier, which is selected from water, especially water for injection, sodium chloride, sodium phosphate, sodium acetate, sodium carbonate, citrate, glycine, glycylglycine, histidine, lysine, arginine, TRIS, sodium citrate, Ringer solution, dextrose, mannite, trehalose, saccharose, sorbite, fructose, maltose, lactose or dextrane, Hank solution, fixed oils, ethyl oleate, substances, which improve the isotonicity and the chemical stability, stabilizing agents, pharmaceutically acceptable proteins, polysaccharides, polylactic acids, polyglycolic acids, polymeric amino acids and amino acid copolymers.

9. The composition according to any one of claims 1 to 8, **characterized in that** it comprises, independently of each other,
- the peptide in an amount of 1 µg to 10 g, preferably of 10 µg to 1 g, in particular of 1 mg to 100 mg, and
- the inhibitor of viral neuraminidase in an amount of 1 µg to 10 g, preferably of 100 µg to 1 g, in particular of 1 mg to 200 mg.

10. The composition according to any one of claims 1 to 9, **characterized in that** it is in a liquid form and comprises, independently of each other,
- the peptide in an amount of 1 µg to 10 g, preferably of 10 µg bis 1 g, in particular of 1 mg to 100 mg, and
- the inhibitor of viral neuraminidase in an amount of 1 µg to 10 g, preferably of 100 µg to 1 g, particularly preferred of 1 mg to 200 mg,
and is provided in a volume of 0.5 to 10 ml, in particular in a volume of 1 to 5 ml.

11. The composition according to any one of claims 1 to 10, **characterized in that** said peptide and/or said inhibitor of viral neuraminidase is provided in a nebulizable powder formulation or in a nebulizable liquid formulation.

12. A set, comprising the following components, each provided in separate containers:
- a peptide, which consists of 7-17 adjacent amino acids and comprises the hexamer TX₁EX₂X₃E, wherein X₁, X₂ and X₃ can be any natural or non-natural amino acid, wherein the peptide has no TNF receptor binding activity and is cyclized, and
- an inhibitor of viral neuraminidase.

13. The set according to claim 12, **characterized in that** said components, each provided in separate containers, are defined as claimed in any one of claims 1 to 11.

14. A composition, comprising
- a peptide, which consists of 7-17 adjacent amino acids and comprises the hexamer TX₁EX₂X₃E, wherein X₁, X₂ and X₃ can be any natural or non-natural amino acid, wherein the peptide has no TNF receptor binding activity and is cyclized, and
- an inhibitor of viral neuraminidase,
for use in preventing and treating influenza.

15. A composition, comprising
- a peptide, which consists of 7-17 adjacent amino acids and comprises the hexamer TX₁EX₂X₃E, wherein X₁, X₂ and X₃ can be any natural or non-natural amino acid, wherein the peptide has no TNF receptor binding activity and is cyclized, and
- an inhibitor of viral neuraminidase,
for use in a pulmonary inflammation, which is caused by influenza.

16. A set, comprising the following components, each provided in separate containers:
- a peptide, which consists of 7-17 adjacent amino acids and comprises the hexamer TX₁EX₂X₃E, wherein X₁, X₂ and X₃ can be any natural or non-natural amino acid, wherein the peptide has no TNF receptor binding activity and is cyclized, and
- an inhibitor of viral neuraminidase,
for use in preventing and treating influenza.

## Revendications

1. Composition, comprenant
- un peptide, qui consiste en 7 à 17 acides aminés voisins et qui comprend l'hexamère TX₁EX₂X₃E, dans lequel X₁, X₂, X₃ peuvent être n'importe quel acide aminé naturel ou non naturel, le peptide ne présentant pas d'activité de liaison de récepteur TNF et est cyclisé, et
- un inhibiteur de neuraminidase virale.

2. Composition selon la revendication 1, dans laquelle le peptide consiste en 7 à 17 acides aminés voisins et comprend l'hexamère TPEGAE.

3. Composition selon la revendication 1 ou 2, dans laquelle le peptide cyclique consiste en une séquence d'acides aminés consécutifs, choisie parmi le groupe constitué de QRETPEGAEAKPWY, PKDTPEGAELKPWY, CGQRETPEGAEAKPWYC, CGPKDTPEGAELKPWYC, et de fragments de ceux-ci comptant au moins 7 acides aminés, les fragments contenant l'hexamère TPEGAE.

4. Composition selon l'une quelconque des revendications 1 à 3, **caractérisée en ce que** le peptide comprend la séquence d'acides aminés CGQRETPEGAEAKPWYC et est cyclisé via les résidus C.

5. Composition selon l'une quelconque des revendications 1 à 4, **caractérisée en ce que** le peptide est cyclisé par un pont disulfure entre les résidus C.

6. Composition selon l'une quelconque des revendications 1 à 5, **caractérisée en ce que** l'inhibiteur de neuraminidase virale est le zanamivir ou l'oseltamivir.

7. Composition selon l'une quelconque des revendications 1 à 6, **caractérisée en ce qu'**elle comprend un support pharmaceutiquement acceptable et est préparée sous forme d'une composition pharmaceutique propre à être administrée à des patients humains.

8. Composition selon l'une quelconque des revendications 1 à 7, **caractérisée en ce qu'**elle comprend un support pharmaceutiquement acceptable choisi parmi l'eau, en particulier l'eau pour des injections, le sel de cuisine, le phosphate de sodium, l'acétate de sodium, le carbonate de sodium, le citrate, la glycine, la glycylglycine, l'histidine, la lysine, l'arginine, le tris-hydroxyméthylène-aminométhane, le citrate de sodium, le liquide de Ringer, le dextrose, le mannitol, le tréhalose, le saccharose, le sorbitol, le fructose, le maltose, le lactose ou le dextrane, la solution de Hank, les huiles fixées, l'oléate d'éthyle, des substances qui améliorent l'isotonie et la stabilité chimiques, des agents de conservation, des protéines pharmaceutiquement acceptables, des polysaccharides, des acides polylactiques, des acides polyglycoliques, des acides aminés polymères et des copolymères d'acides aminés.

9. Composition selon l'une quelconque des revendications 1 à 8, **caractérisée en ce qu'**elle comporte - indépendamment l'un de l'autre -
- le peptide, en une quantité de 1 µg à 10 g, de préférence de 10 µg à 1 g, en particulier de 1 mg à 100 mg, et
- l'inhibiteur de neuraminidase virale, en une quantité de 1 µg à 10 g, de préférence de 100 µg à 1 g, en particulier de 1 mg à 200 mg.

10. Composition selon l'une quelconque des revendications 1 à 9, **caractérisée en ce qu'**elle se présente sous forme liquide et qu'elle comporte - indépendamment l'un de l'autre -
- le peptide, en une quantité de 1 µg à 10 g, de préférence de 10 µg à 1 g, en particulier de 1 mg à 100 mg, et
- l'inhibiteur de neuraminidase virale, en une quantité de 1 µg à 10 g, de préférence de 100 µg à 1 g, en particulier de 1 mg à 200 mg,
et qu'elle se présente en un volume de 0,5 à 10 ml, en particulier en un volume de 1 à 5 ml.

11. Composition selon l'une quelconque des revendications 1 à 10, **caractérisée en ce que** le peptide et/ou l'inhibiteur de neuraminidase virale se présentent dans une formulation pulvérulente nébulisable ou une formulation liquide nébulisable.

12. Kit, comprenant les composants suivants, prévus respectivement dans des récipients séparés les uns des autres :
- un peptide, qui consiste en 7 à 17 acides aminés voisins et qui comprend l'hexamère TX₁EX₂X₃E, dans lequel X₁, X₂, X₃ peuvent être n'importe quel acide aminé naturel ou non naturel, sachant que le peptide ne présente pas d'activité de liaison de récepteur TNF et est cyclisé, et
- un inhibiteur de neuraminidase virale.

13. Kit selon la revendication 12, **caractérisé en ce que** les composants prévus respectivement dans des récipients séparés les uns des autres sont définis comme dans une quelconque des revendications 1 à 11.

14. Composition, comprenant
- un peptide, qui consiste en 7 à 17 acides aminés voisins et qui comprend l'hexamère TX₁EX₂X₃E, dans lequel X₁, X₂, X₃ peuvent être n'importe quel acide aminé naturel ou non naturel, sachant que le peptide ne présente pas d'activité de liaison de récepteur TNF et est cyclisé, et
- un inhibiteur de neuraminidase virale,
destinée à être appliquée pour empêcher et pour soigner la grippe (influenza).

15. Composition, comprenant
- un peptide, qui consiste en 7 à 17 acides aminés voisins et qui comprend l'hexamère TX₁EX₂X₃E, dans lequel X₁, X₂, X₃ peuvent être n'importe quel acide aminé naturel ou non naturel, sachant que le peptide ne présente pas d'activité de liaison de récepteur TNF et est cyclisé, et
- un inhibiteur de neuraminidase virale,
destinée à être appliquée dans le cas d'une pneumonie provoquée par la grippe.

16. Kit, comprenant les composants suivants, prévus respectivement dans des récipients séparés les uns des autres :
- un peptide, qui consiste en 7 à 17 acides aminés voisins et qui comprend l'hexamère TX₁EX₂X₃E, dans lequel X₁, X₂, X₃ peuvent être n'importe quel acide aminé naturel ou non naturel, sachant que le peptide ne présente pas d'activité de liaison de récepteur TNF et est cyclisé, et
- un inhibiteur de neuraminidase virale.
destiné à être appliqué pour empêcher et pour soigner la grippe (influenza).
